# EUROPÄISCHE PATENTSCHRIFT

(11) **EP 1 247 446 B1**
(45) Veröffentlichungstag und Bekanntmachung des Hinweises auf die Patenterteilung: **03.01.2007**
(21) Anmeldenummer: 01107795.5
(22) Anmeldetag: 05.04.2001
(51) Int. Cl.: A01M 1/20, A61L 9/03

(54) **Vorrichtung zum Verdampfen von flüchtigen Substanzen, insbesondere von Insektiziden und/oder Duftstoffen**
Device for vaporising fluids, particularly insecticides and/or perfumes
Dispositif de vaporisation de substances liquides, notamment insecticides et/ou parfums

(43) Veröffentlichungstag der Anmeldung: 09.10.2002
(73) Patentinhaber: C.T.R., Consultoria, Técnica e Representaçoes Lda, 2715-251 Almargem do Bispo (PT)
(72) Erfinder: Vieira, Pedro Queiroz, 2775 - 178 Parede (PT)
(74) Vertreter: Liebl, Thomas

(56) Entgegenhaltungen:
- EP-A- 0 689 766
- EP-A- 0 911 041
- EP-A- 1 055 430
- WO-A-01/05442
- PATENT ABSTRACTS OF JAPAN vol. 1997, no. 05, 30. Mai 1997 (1997-05-30) & JP 09 010292 A (CASIO COMPUT CO LTD), 14. Januar 1997 (1997-01-14)
- PATENT ABSTRACTS OF JAPAN vol. 1996, no. 10, 31. Oktober 1996 (1996-10-31) & JP 08 155018 A (CASIO COMPUT CO LTD), 18. Juni 1996 (1996-06-18)

## Beschreibung

Die Erfindung betrifft eine Vorrichtung zum Verdampfen von flüchtigen Substanzen, insbesondere von Insektiziden und/oder Duftstoffen, nach dem Oberbegriff des Anspruchs 1.

Derartige Vorrichtungen zum Verdampfen sind allgemein bekannt. So sind beispielsweise Verdampfungsvorrichtungen bekannt, bei denen ein in eine Verdampfungsvorrichtung eingesetztes und mit einem Wirkstoff imprägniertes Plättchen erhitzt wird, um den Wirkstoff zu verdampfen. Weiter ist es auch bekannt, in ein Gehäuse einer Verdampfungsvorrichtung einen eine flüchtige Substanz enthaltenden Behälter einzusetzen. Dieser Behälter umfasst einen Docht, der die zu verdampfende Substanz mittels der Kapillarwirkung aus dem Behälter fördert, wobei das aus dem Behälter ragende Dochtende benachbart zu einem Heizelement, wie z. B. einem Keramikblock, angeordnet ist, so dass die Substanz durch die vom Keramikblock abgestrahlte Wärme verdampft und über Lüftungsschlitze im Gehäuse aus diesem in die Umgebung entweichen kann.

Oftmals ist es jedoch auch wünschenswert, dass mit Verdampfungsvorrichtungen eine Anpassung der Verdampfungsleistung und damit des Verdampfungsgrades an die jeweils vorherrschenden Raumbedingungen bzw. an unterschiedliche Sensitivitäten von sich im Raum befindlichen Personen möglich ist.

So ist es z. B. bei kleinen Räumen mit ungenügender Luftzufuhr grundsätzlich wünschenswert, dass der Verdampfungsgrad im Gegensatz zu großen und ggf. gut belüfteten Räumen niedriger einstellbar ist, was mit derartigen Verdampfungsvorrichtungen jedoch nicht möglich ist. Des weiteren ist Insbesondere hinsichtlich der Verdampfung von Insektiziden eine Einstellbarkeit wünschenswert, damit der Verdampfungsgrad individuell entsprechend der Sensitivität von sich im Raum befindlichen Personen eingestellt werden kann. Auch dies ist mit derartigen Vorrichtungen nicht möglich.

Vorrichtungen, mit denen der Verdampfungsgrad eingestellt werden kann, sind bereits allgemein gekannt. So beispielsweise aus der EP 0 962 132 A1 oder aus der WO 98/19526, die relativ aufwendig und teuer in der Herstellung sind.

Dies trifft auch auf die Verdampfungsvorrichtungen der EP 0 943 344 A1 und der WO 98/58692 zu, denen jeweils die Aufgabe zugrunde liegt, durch Relativverstellung des Dochtes zum Heizblock die Verdampfungsleistung und damit den Verdampfungsgrad zu ändern.

Weiter aus der gattungsbildenden EP 0911041 A2 eine Verdampfungsvorrichtung bekannt, die eine Heizeinrichtung mit zwei Widerstandselementen als Heizelemente aufweist. Diese beiden Widerstandselemente sind beabstandet zu einem Docht in einer aus zwei Hälften zusammensetzbaren Haube aus Kunststoff gelagert, mittels der über eine entsprechende geometrische Auslegung der Haube ein Luftstrom mit hoher Temperatur zum Docht gelenkt wird. Mittels der Haube soll eine solche gezielte Lagerung und Beabstandung der Widerstandselemente vom Docht erzielt werden, dass ein direkter Kontakt der Widerstandselemente mit der Dochtoberfläche zur Vermeidung von Ablagerungen der zu verdampfenden Substanz auf den Widerständen vermieden wird. Ein derartiger Aufbau in einer Heizeinrichtung ist relativ bauteilintensiv und zudem aufwendig in der Montage, was die Vorrichtung insgesamt relativ teuer macht. Die Widerstände werden über eine Timer-Einrichtung gleichzeitig angesteuert, so dass diese nur zusammen aktiviert bzw. deaktiviert sind, da die Verdampfungszeit über die Timer-Einrichtung selektiv, d. h. lediglich zeitweise vorgegeben ist.

Aus der WO 01/05442 A1und aus der JP 08 15 50 18 A ist jeweils eine Verdampfungsvorrichtung bekannt, bei der in Verbindung mit zwei unterschiedlichen Heizelementen zwei separate und voneinander beabstandete Heizblöcke vorgesehen sind. Ein derartiger Aufbau ist relativ bauteilintensiv und damit aufwendig und teuer inder Montage und der Herstellung. Die EP 0689766 A zeigt ebenfalls einen Aufbau mit zwei Widerstandselementen aus Heizelementen, die als separate Bauteile auf einer Platte montiert sind.

Die JP 09010292 A zeigt ferner eine Vorrichtung mit einem Schwenkventil zum selektiven Öffnen und Schließen unterschiedlicher Verdampfungszellen.

Schließlich zeigt die EP 1055430 A einen Heizblock mit lediglich einem darin integrierten Widerstandselement.

Aufgabe der Erfindung ist es, eine alternative Vorrichtung zum Verdampfen von flüchtigen Substanzen, insbesondere von Insektiziden und/oder Duftstoffen zu schaffen, mit der die Verdampfungsleistung und damit der Verdampfungsgrad einfach einstellbar ist und die zudem ohne großen Bauteilaufwand relativ einfach und preiswert herstellbar ist.

Diese Aufgabe wird gelöst mit den Merkmalen des Anspruch 1.

Gemäß Anspruch 1 sind in den Heizblock wenigstens zwei Heizelemente eingegossen, mit denen unterschiedliche Heizleistungen einstellbar sind, wobei die wenigstens zwei Heizelemente zu deren Aktivierung und/oder Deaktivierung mit der gleichzeitig die Einstelleinrichtung bildenden Schalteinrichtung gekoppelt sind.

Mit einem derartigen kompakten und durch das Ver- bzw. Eingießen der Heizelemente wenig bauteilintensiven Aufbau kann über eine einzige Schalteinrichtung, die in einer Doppelfunktion gleichzeitig die Einstelleinrichtung ausbildet, vorteilhaft je nach der Anzahl der vorgesehenen Heizelemente der jeweils gewünschte Verdampfungsgrad, d. h. die jeweilige Verdampfungsleistung eingestellt werden, in dem der Verdampfungsgrad einfachst durch Veränderung der Heizleistung, z. B. durch Umschalten zwischen den einzelnen Heizelementen, eingestellt wird. Durch die Möglichkeit der Integration der einzelnen Heizelemente in einen Heizblock ist die Vorrichtung trotz der Anordnung mehrerer Heizelemente nach wie vor kompakt und in gewünschter Weise kleinbauend herstellbar.

Beispielsweise können durch unterschiedliche Heizleistungen unterschiedlicher Heizelemente die Verdampfungsleistungen so reguliert werden, dass in Abhängigkeit von der zu verdampfenden Substanz, Duftstoff oder Insektizid, die Verdampfung schnell oder langsam erfolgt.

Insgesamt kann somit die Verdampfungsleistung mit einem derartigen Aufbau auf besonders einfache Weise gut auf die gerade zu verdampfende Substanz abgestimmt werden, wobei aufgrund der Funktionsintegration an einem einzigen Bauteil ein sehr vielfältiger multifunktioneller Einsatz desselben möglich ist.

Je nach der Anzahl der am Heizblock angeordneten Heizelemente können diese alle oder wenigstens zum Teil eine unterschiedliche Heizleistung aufweisen, so dass je nach der Anzahl der aktivierten Heizelemente der Verdampfungsgrad einstellbar ist. Weiter sind gemäß einer besonders bevorzugten Ausgestaltung nach Anspruch 2 die Einsatzmöglichkeiten der erfindungsgemäßen Vorrichtung wesentlich erhöht, so dass eine noch bessere Abstimmung des Verdampfungsgrades auf die zu verdampfende Substanz möglich ist.

Ein besonders kompakter Aufbau wird mit den Merkmalen des Anspruchs 3 erzielt. Bei einem solchen bevorzugten, konkreten Aufbau einer Vorrichtung können über die gleichzeitig als Einstelleinrichtung ausgebildete Schalteinrichtung entweder beide Heizelemente deaktiviert sein oder aber auch je nach der gewünschten Verdampfungsleistung ggf. in Abhängigkeit von der zu verdampfenden Substanz entweder das eine oder das andere Heizelement zugeschalten und damit aktiviert sein. Ggf. können auch in einer Schaltstellung beide Heizelemente gemeinsam aktiviert sein. Mit einem derartigen kompakten Aufbau, bei dem zwei Heizelemente vorgesehen sind, ergeben sich somit eine Mehrzahl von Einsatz- und Verwendungsmöglichkeiten mittels einer einzigen Vorrichtung zum Verdampfen von flüchtigen Substanzen, so dass eine einfache Anpassung und Umstellung auf die jeweiligen Einsatzfälle sehr schnell möglich ist.

Bei einer Weiterbildung nach Anspruch 4 ergibt sich am Heizblock eine besonders gut kontrollierbare und einstellbare Wärmeleitung in Richtung zu der Dochtaussparung hin, mit der eine optimale Verdampfung der flüchtigen Substanz möglich ist.

Eine besonders gut kontrollierbare, einfache Einstellbarkeit insbesondere hinsichtlich der unterschiedlichen Verdampfungsleistungen bei unterschiedlichen Heizelementen ergibt sich mit den Merkmalen des Anspruchs 5.

Mit einer Ausführungsform gemäß Anspruch 6 ergibt sich eine noch höhere Flexibilität und eine noch bessere Funktionsintegration dadurch, dass je nach der Anzahl der Heizelemente und der entsprechend zugeordneten Dochtaussparungen eine Vielzahl von einzelnen Heizeinheiten jeweils bestehend aus Heizelement und Dochtaussparung in einer einzigen Vorrichtung integriert werden können. Über die Schalteinrichtung können diese einzelnen Heizeinheiten jeweils zusammen oder einzeln aktiviert und deaktiviert werden, so dass eine individuelle Zu- und Abschaltung je nach den gerade gegebenen Verdampfungssituationen und wünschen möglich ist.

Grundsätzlich kann jeweils ein Heizelement einer Dochtaussparung zugeordnet sein. Jedoch ist es auch möglich, dass einer der mehreren Dochtaussparungen mehr als ein Heizelement, z.B. mit unterschiedlichen Heizleistungen, zugeordnet ist, so dass ein und demselben Dochtaussparungsbereich je nach gerade aktiviertem zugeordneten Heizelement unterschiedliche Verdampfungsleistungen, z.B. schnell oder langsam, zuordenbar sind. Ggf. können auch alle zugeordneten Heizelemente gemeinsam aktiviert sein oder aber auch bloß einzelne von mehreren, z.B. paarweise, aktiviert sein. Ebenso ist es aber auch möglich, dass einem von mehreren Heizelementen mehrere Dochtaussparungen zugeordnet sind, so dass ggf. über dieses Heizelement mehrere Dochtaussparungsbereiche auf Verdampfungstemperatur erwärmt werden können. Insgesamt kann somit die Verdampfungsleistung mit einem derartigen Aufbau auf besonders einfache Weise gut auf die gerade zu verdampfende Substanz abgestimmt werden, wobei aufgrund der Funktionsintegration an einem einzigen Bauteil ein sehr vielfältiger multifunktioneller Einsatz desselben möglich ist. Grundsätzlich ist hier bei diesem speziellen Aufbau aber auch der Einsatz von gleichen Heizelementen, d.h. von eine gleiche Heizleistung erzeugenden Heizelementen möglich, wenn z.B. unterschiedliche Substanzen verdampft werden sollen, die in etwa eine gleiche Verdampfungstemperatur aufweisen.

Gemäß einer bevorzugten Ausgestaltung nach Anspruch 7 sind am Heizblock zwei Dochtaussparungen sowie zwei Heizelemente angeordnet, wobei jeweils ein Heizelement einer Dochtaussparung zugeordnet ist. Grundsätzlich gibt es verschiedene Möglichkeiten die beiden Dochtaussparungen sowie die entsprechend zugeordneten beiden Heizelemente am Heizblock anzuordnen. Um jedoch die einzelnen Heizeinheiten bestehend aus Heizelement und entsprechend zugeordneter Dochtaussparung separat voneinander aktivieren und deaktivieren zu können, ohne dass es durch die eine Heizeinheit auch zu einer Aufheizung des Bereichs der anderen Heizeinheit, insbesondere des Durchgangslochbareichs, auf die dortige Verdampfungstemperatur kommt, wird gemäß Anspruch 8 vorgeschlagen, dass zwei Dochtaussparungen beabstandet voneinander sowie bezogen auf eine Draufsicht auf den Heizblock in einem mittleren Bereich zwischen zwei vorzugsweise im randseitigen Bereich des Heizblocks angeordneten Heizelementen angeordnet sind. Mit einer derartigen konkreten Anordnung der Heizelemente im ausreichenden Abstand von der anderen Heizeinheit und insbesondere von der anderen Dochtaussparung wird auf einfache Weise erreicht, dass keine Wärmeleitung in den Bereich der anderen Heizeinheit bzw. der anderen Dochtaussparung dergestalt erfolgen kann, dass der dortige Bereich auf eine Verdampfungstemperatur einer dort eventuell vorhandenen und nicht zu Verdampfen gewünschten flüchtigen Substanz erwärmt wird.

Grundsätzlich können gemäß Anspruch 9 die beiden Behälter jeweils separate Behältnisse sein, die jeweils einen einzigen Docht aufweisen. Alternativ dazu ist jedoch ebenfalls nach Anspruch 9 auch ein einziges Behältnis mit zwei voneinander abgetrennten Kammern als Behälter möglich, wobei in den beiden als Kammern ausgebildeten Behältern z. B. unterschiedliche zu verdampfende Substanzen aufgenommen sein können, in die jeweils ein Docht ragt. Insbesondere im letzteren Fall ergibt sich ein besonders kompakter Aufbau der Verdampfungsvorrichtung im praktischen Einsatz.

Die thermische Entkopplung der unterschiedlichen Heizeinheiten wird mit den Maßnahmen des Anspruchs 10 noch erheblich verstärkt. Dieses dort vorgesehene Trennmittel ist gemäß Anspruch 11 bevorzugt durch einen wenigstens im Bereich zwischen den beiden Dochtaussparungen durch den Heizblock durchgehenden Luftspalt gebildet. Eine derartige thermische Entkopplung mittels Trennmittel wie beispielsweise des Luftspaltes ist grundsätzlich auch bei Verdampfungsvorrichtungen möglich, bei denen mehr als zwei Heizeinheiten vorgesehen sind.

Ein kleinbauendes und gut geeignetes Heizelement mit einer guten Heizleistung wird gemäß Anspruch 12 durch ein elektrisches Widerstandselement gebildet, das im Heizblock aufgenommen ist. Gemäß Anspruch 13 sind die elektrischen Widerstandselemente in etwa stabförmig ausgebildet sowie insbesondere in Verbindung mit zwei Widerstandselementen mit mittiger Dochtaussparung in etwa parallel zueinander ausgerichtet. Dadurch ergibt sich ein besonders kompakter Aufbau einer Heizeinrichtung.

Zur Bereitstellung unterschiedlicher Heizleistungen können gemäß Anspruch 14 im Falle des Einsatzes von Widerstandselementen als Heizelemente diese unterschiedliche Widerstandswerte aufweisen.

Mit den Maßnahmen gemäß Anspruch 15 ist ein einfacher und kompakter Aufbau der Vorrichtung insgesamt möglich, wobei zudem ein beliebiger Einsatz in unterschiedlichen Räumen bzw. Gebäuden etc. möglich ist.

Grundsätzlich kann das elektrische Widerstandselement durch jedes bekannte Widerstandselement gebildet sein, z. B. durch PTC-Widerstandselemente. Eine besonders bevorzugte Ausgestaltung des Widerstandselementes ist jedoch mit Anspruch 16 beansprucht.

Vorteilhaft kann ein derartiges Widerstandselement für Heizeinrichtungen von Verdampfungsvorrichtungen relativ kleinbauend ausgebildet werden, so dass auch der Heizblock und die Heizeinrichtung und damit auch das die Heizeinrichtung aufnehmende Gehäuse insgesamt relativ kleinbauend ausgebildet werden können. Dadurch können beim gleichzeitigen Einsatz eines oder auch von zweien oder mehreren entsprechend angepassten kleinvolumigen Behältern in das Gehäuse Verdampfungsvorrichtungen mit geringen Abmessungen, d. h. eine miniaturisierte Verdampfungsvorrichtung geschaffen werden. Eine derartige miniaturisierte Verdampfungsvorrichtung ist aufgrund des reduzierten Material- und Bauteilaufwands zudem auch relativ einfach und damit preiswert herstellbar, z. B. als einmal benutzbarer Wegwerfartikel.

Ein weiterer besonderer Vorteil einer derartigen Verdampfungseinrichtung ist, dass die Verdampfungstemperatur mit einem derartigen Widerstandselement, bei der die Widerstandsschicht zur Einstellung eines bestimmten Widerstandswertes bereichsweise eingeschnitten und/oder bereichsweise eingeschliffen ist, optimal auf die Zusammensetzung der jeweils zu verdampfenden Substanz abgestimmt werden kann. Dadurch kann z. B. auch vorteilhaft die Gefahr einer Entflammbarkeit der Vorrichtung insgesamt durch bestimmte Bestandteile reduziert und zudem auch eine ggf. negative Auswirkung auf den Verdampfungsgrad vermieden werden.

Grundsätzlich gibt es verschiedene Möglichkeiten die Widerstandsschicht zur Einstellung eines bestimmten Widerstandswertes einzuschneiden bzw. einzuschleifen. Gemäß einer bevorzugten Ausführungsform nach Anspruch 17 wird die Widerstandsschicht um den stabförmigen, vorzugsweise zylindrischen Widerstandskörper herum spiralförmig eingeschnitten, vorzugsweise durch Laserspiralschneiden. Mit einem derartigen spiralförmigen Einschnitt kann der Widerstandswert für eine optimale Verdampfungsleistung auf besonders einfache Weise sehr genau eingestellt werden. Die Widerstandsschicht kann grundsätzlich ebenfalls aus unterschiedlichen Materialien hergestellt sein, so z. B in Form einer Edelmetallschicht. Bevorzugt ist die Widerstandsschicht jedoch als Metalloxidschicht ausgebildet, vorzugsweise als Nickel-Chromlegierungsschicht, die vorteilhaft thermochemisch aufgebrannt, z. B. als Dünnschicht im Vakuum aufgedampft bzw. gesputtert, wird. Nach der Aufbringung der Widerstandsschicht wird diese vorzugsweise einem thermischen Verfahren unterworfen, um die Widerstandsschicht zu stabilisieren. Der Widerstandskörper kann dabei aus Keramik hergestellt sein, vorzugsweise mit einem hohen Gehalt an Al₂O₃ (Aluminiumoxid), wodurch eine besonders gute Wärmeleitfähigkeit des Widerstandskörpers und damit des Widerstandselementes insgesamt erreichbar ist. Der Gehalt an Al₂O₃ ist abhängig von den jeweils konkret gegebenen Einbauverhältnissen, z. B. dem verwendeten Gehäusematerial, Dochtmaterial etc.

Auf die Enden des beschichteten stabförmigen Widerstandskörpers sind Metallkappen aufsetzbar, die vorzugsweise aufgepresst sind. An diesen Kappen ist jeweils eine elektrische Leitung angebracht, vorzugsweise angeschweißt, die jeweils mit dem Anschlussstecker gekoppelt sind. Vorzugsweise werden als elektrische Leitungen für eine gute elektrische Leitung Kupferdrähte verwendet. In derartigen Metallkappen wird zudem ein guter elektrischer Kontakt zu der Widerstandsschicht auf einfache und funktionssichere Weise hergestellt.

Grundsätzlich gibt es verschiedene Möglichkeiten, das stabförmige Widerstandselement am Heizblock anzuordnen. In einer besonders bevorzugten Ausführungsform nach Anspruch 18 ist vorgesehen, dass das stabförmige Widerstandselement in eine Aussparung des Heizblocks einbringbar ist, wobei das Widerstandselement dort mit einem eine hohe Wärmeleitfähigkeit aufweisenden Material vergossen ist, um das Widerstandselement im Heizblock sicher zu fixieren. Das eine hohe Wärmeleitfähigkeit aufweisende Material ist vorzugsweise ein flammenbeständiger Isolationszement. Weiter ist an gegenüberliegenden Aussparungsenden der Aussparung zu beiden Seiten des Widerstandselementes vorzugsweise jeweils ein Schlitz ausgebildet, durch die die elektrischen Leitungen aus dem Heizblock zu dem Anschlussstecker hin herausgeführt sind. Mit einem derartigen Aufbau kann das Widerstandselement im Rahmen der Montage auf einfache Weise in die Aussparung eingesetzt werden, z. B. auch mit einem Klemmschluss, so dass das Widerstandselement beim Vergießen nicht mehr verrutschen kann. Weiter können die elektrischen Leitungen mit einem derartigen Aufbau auf einfache Weise auch in Richtung zu dem Anschlussstecker hin gebogen werden. Die elektrischen Leitungen können dabei auf herkömmliche Weise isoliert sein.

Gemäß Anspruch 19 ist die Dochtaussparung als vorzugsweise rundes Durchgangsloch im Heizblock ausgebildet.

Je nach Einsatzzweck der Vorrichtung gibt es nach Anspruch 20 verschiedene Möglichkeiten die Schalteinrichtung auszubilden. Diese kann insbesondere in Verbindung mit einem kompakten, kleinbauenden Gerät direkt an diesem, z. B. am Gehäuse, integriert sein und als Handschalter ausgebildet sein. Alternativ dazu ist es aber auch möglich, die Schalt- und/oder Steuereinrichtung durch einen programmierbaren Mikroprozessor auszubilden, der entsprechend mit der Vorrichtung bzw. mit dem Gehäuse gekoppelt ist.

Eine besonders einfache und schnelle Montage der Heizeinrichtung im Gehäuse ermöglicht ein Aufbau gemäß Anspruch 21. Die Oberschale und die Unterschale können beispielsweise durch Rast- und/oder Clipelemente miteinander verbunden werden. In der Unterschale sind vorzugsweise Verbindungsmittel zur Verbindung eines Behälters mit dem Gehäuse ausgebildet, z. B Rastelemente. Wenigstens eine der beiden Schalen weist dabei auch wenigstens einen Lüftungsschlitz zum Entweichen der verdampften Substanz in die Umgebung auf. Die Lüftungsschlitze sind dabei vorzugsweise im Bereich oberhalb des Dochtendes in der Oberschale ausgebildet. Ein derartiges zweiteiliges Gehäuse ist auf besonders einfache und preiswerte Weise ohne große Bauteilvielfalt herstellbar.

Die Erfindung wird nachfolgend anhand einer Zeichnung näher erläutert.

Es zeigen:
- Fig. 1: eine schematische Draufsicht auf eine Heizeinrichtung einer Vorrichtung zum Verdampfen von flüchtigen Substanzen,
- Fig. 2: eine schematische, perspektivische Darstellung der Ansicht gem. Fig. 1,
- Fig. 3-5: schematische Darstellungen des Zusammenbaus der Verdampfungsvorrichtung in unterschiedlichen Montagezeitpunkten,
- Fig. 6: eine schematische Draufsicht auf ein Widerstandselement,
- Fig. 7: eine schematische Schnittansicht entlang der Linie A-A der Fig. 1,
- Fig. 8: eine schematische, perspektivische Darstellung einer alternativen Ausführungsform einer Heizeinrichtung für eine Vorrichtung zum Verdampfen von flüchtigen Substanzen,
- Fig. 9: eine schematische Draufsicht auf einen Heizblock der Ausführungsform gem. Figur 8,
- Fig. 10: ein schematische Schnittansicht entlang der Linie B-B der Fig. 9 und
- Fig. 11-13: schematische, perspektivische Darstellungen des Zusammenbaus der Vorrichtung zum Verdampfen von flüchtigen Substanzen gem der alternativen Ausführungsform zu unterschiedlichen Montagezeitpunkten.

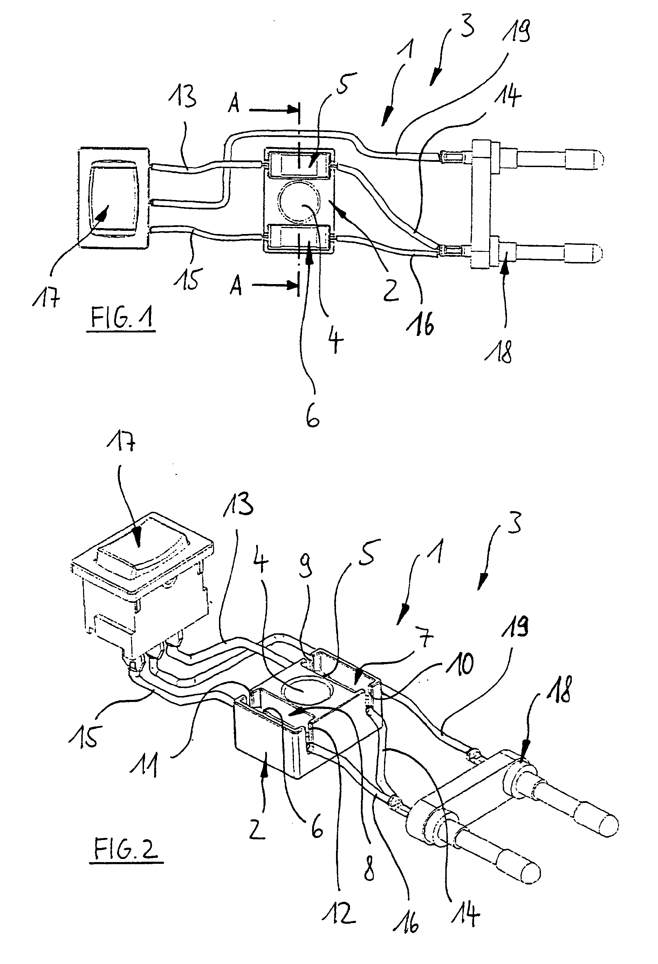

In den Figuren 1 und 2 sind schematische Darstellungen in unterschiedlichen Ansichten einer Heizeinrichtung 1 mit einem Heizblock 2 als Bestandteil einer hier nicht im Detail dargestellten Verdampfungsvorrichtung 3 gezeigt.

Der Heizblock 2 weist in der Draufsicht gemäß Fig. 1 eine in etwa rechteckige Form auf, wobei in etwa in einem mittleren, zentralen Bereich des Heizblocks 2 eine Dochtaussparung 4 ausgebildet ist. Diese Dochtaussparung 4 liegt in etwa in einem mittleren Bereich zwischen zwei als elektrische Widerstandselemente 5, 6 ausgebildeten Heizelementen.

Diese Widerstandselemente 5, 6 sind, wie dies insbesondere aus der Fig. 2 ersichtlich ist, in einer Aussparung 7, 8 am Heizblock 2 aufgenommen und werden dort, was hier allerdings nicht dargestellt ist, mit einem eine hohe Wärmeleitfähigkeit aufweisenden Material, z. B. einem flammenbeständigen Isolationszement, vergossen.

An den gegenüberliegenden Aussparungswänden ist zu beiden Seiten der Widerstandselemente 5, 6 jeweils ein Schlitz 9, 10, 11, 12 ausgebildet, durch die mit den Widerstandselementen 5, 6 verbundene elektrische Leitungen 13, 14, 15 und 16 herausgeführt sind. In der Fig. 7 ist ein schematischer Querschnitt durch den Heizblock 2 entlang der Linie A-A der Fig. 1 gezeigt.

Die elektrische Leitung 13 ist vom Widerstandselement 5 ausgehend und die elektrische Leitung 15 ist vom Widerstandselement 6 ausgehend jeweils zu einem Handschalter 17 geführt, während die elektrische Leitung 14 des Wider standselements 5 und die elektrische Leitung 16 des Widerstandselements 7 jeweils zu einem Anschlussstecker 18 geführt sind. Ferner ist der Handschalter 17 noch mit einer elektrischen Leitung 19 mit dem Anschlussstecker 18 verbunden.

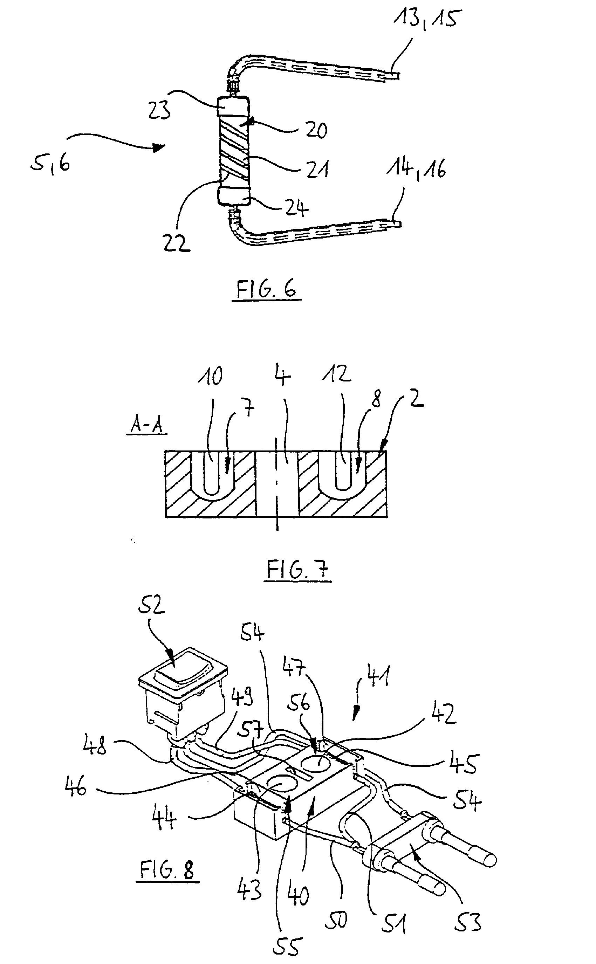

In der Fig. 6 ist der Aufbau der Widerstandselemente 5, 6 in vergrößerter Darstellung gezeigt. So umfassen die Widerstandselemente 5, 6 jeweils einen stabförmigen Widerstandskörper 20, der vorzugsweise aus Keramik hergestellt ist mit einem bestimmten Gehalt an Al₂O₃ zur Einstellung guter Wärmeleiteigenschaften. Dieser Widerstandskörper 20 ist mit einer Widerstandsschicht 21 aus Metalloxid beschichtet, wobei zur Einstellung eines bestimmten Widerstandswertes die Widerstandsschicht 21 spiralförmig, z. B. durch Laserschneiden eingeschnitten ist, so dass ein Spiraleinschnitt 22 um den stabförmigen, zylindrischen Widerstandskörper 20 herum ausgebildet ist.

Auf die Enden des beschichteten, stabförmigen, zylindrischen Widerstandskörpers 20 ist für eine elektrische Verbindung zu der Widerstandsschicht 21 jeweils eine Metallkappe 23, 24 aufgepresst. An diesen Kappen 23, 24 ist jeweils eine der elektrischen Leitungen 13, 14, 15, 16, vorzugsweise ein Kupferdraht angeschweißt, die mit einem Isolationsmaterial isoliert sind.

Wie dies weiter aus der Fig. 1 entnommen werden kann, sind die Widerstandselemente 5, 6 in etwa parallel zueinander angeordnet und weisen in etwa einen gleichen Abstand zu der als Durchgangsloch ausgebildeten Dochtaussparung 4 auf. Die beiden Widerstandselemente 5, 6 weisen bevorzugt unterschiedliche Widerstandswerte für unterschiedliche Heizleistungen auf.

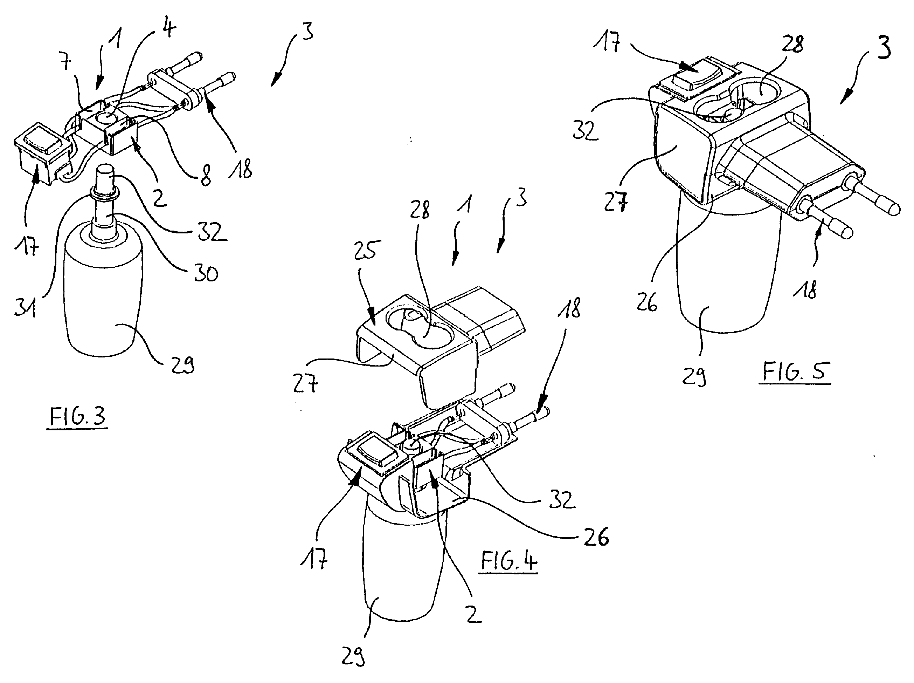

In den Figuren 3 bis 5 wird der Zusammenbau der gesamten Verdampfungsvorrichtung 3 schrittweise näher erläutert. Wie dies insbesondere den Figuren 4 und 5 entnommen werden kann, ist der Heizblock 2 im montierten Zustand in einem Gehäuse 25 aufgenommen, wobei der Heizblock hierzu, wie dies aus der Fig. 4 ersichtlich ist, vorzugsweise mit einer Unterschale 26 des Gehäuses verbunden wird. In dieser Unterschale 26 ist ferner auch eine Aufnahme für eine Integration des Handschalters 17 am Gehäuse 25 vorgesehen. Auf die Unterschale 26 kann dann eine Oberschale 27 aufgeclipst werden, wobei diese Oberschale einen Lüftungsschlitz 28 aufweist, über den die zu verdampfenden Substanzen in die Umgebung entweichen können.

Wie dies insbesondere aus den Figuren 3 und 4 ersichtlich ist, ist in das Gehäuse 25 als weiterer Bestandteil der Verdampfungsvorrichtung 3 ein Behälter 29 einsetzbar, in den ein Docht 30 eingesetzt wird, wobei dieser Docht 30 mit einem Dochthaltering 31 so am Behälter 29 festlegbar ist, dass der Docht 30 mit einem Dochtende 32 aus dem Behälter 29 hervorragt.

Im eingesetzten Zustand des Behälters 29 ragt dieses Dochtende 32, wie dies aus der Fig. 4 ersichtlich ist, in die Dochtaussparung 4 des Heizblocks 2 hinein.

Mit einer derartigen Verdampfungsvorrichtung 3 sind unterschiedliche Betriebszustände einstellbar, so z. B. ein erster Betriebszustand, bei dem durch eine entsprechende Stellung des Handschalters 17 beide Widerstandselemente 5, 6 deaktiviert sind, so dass kein Aufheizen des Heizblocks 2 erfolgt.

Für den Fall, dass das Widerstandselement 5 beispielsweise eine höhere Heizleistung bereitstellt als das Widerstandselement 6, kann z. B. vorgesehen werden, dass bei einer Zuschaltung lediglich des Widerstandselementes 5 eine besonders schnelle Verdampfung der im Behälter 29 aufgenommenen und über die Kapillarwirkung des Dochtes 30 in den Bereich der Dochtaussparung 4 geförderten zu verdampfenden Substanz mit hohem Verdampfungsgrad erfolgt. Alternativ dazu kann bei einer Zuschaltung lediglich des Widerstandselementes 6, das einen geringeren Widerstandswert aufweist, eine entsprechend langsamere Verdampfung der im Behälter 29 aufgenommenen Substanz erfolgen.

Ggf. kann über den Handschalter 17 auch eine derartige Schaltung erfolgen, dass für eine besonders schnelle und effektive Verdampfung beide Widerstandselemente 5, 6 gleichzeitig zugeschaltet sind.

Die Wahl der Schaltstellung bzw. die Wahl des Widerstandselements 5, 6 zum Verdampfen kann dabei in Abhängigkeit von den eingesetzten flüchtigen Substanzen, z. B. Insektiziden und/oder Duftstoffen erfolgen.

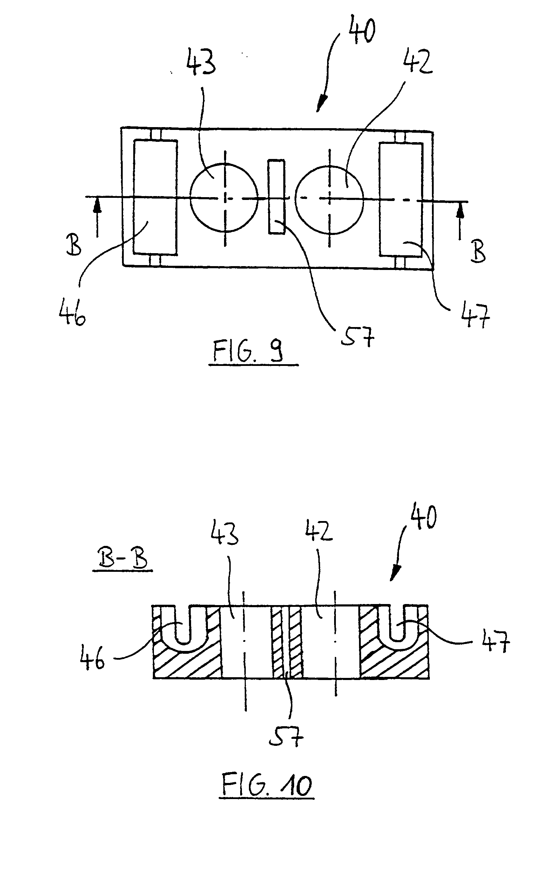

In der Fig. 8 ist eine alternative Ausführungsform eines Heizblocks 40 einer Verdampfungsvorrichtung 41 gezeigt, bei der der Heizblock 40 zwei voneinander beabstandete Dochtaussparungen 42, 43 aufweist, die bezogen auf die in der Fig. 9 dargestellte Draufsicht auf den Heizblock 40 in einem mittleren Bereich desselben zwischen zwei im randseitigen Bereich des Heizblocks angeordneten Widerstandselementen 44, 45 (Fig. 8) angeordnet sind. In der Fig. 10 ist ein Querschnitt entlang der Linie B-B durch den Heizblock 40 der Fig. 9 dargestellt.

Die Widerstandselemente 44, 45 entsprechen vom Aufbau her dem Widerstandselement 3, wie es in Verbindung mit den Figuren 1 bis 7 ausführlich beschrieben worden ist, so dass hierauf nicht mehr näher eingegangen wird.

Die Widerstandselemente 44, 45 sind den Aussparungen 46, 47 am Heizblock 40 aufgenommen und dort einzementiert, wobei jedem Widerstandselement 44, 45 elektrische Anschlussleitungen 48, 49 sowie 50, 51 zugeordnet sind, die durch Schlitze im Bereich der Aussparungsseitenwände herausgeführt sind dergestalt, dass die Anschlussleitung 48 des Widerstandselements 44 sowie die Anschlussleitung 49 des Widerstandselements 45 jeweils zu einem Handschalter 52 geführt sind. Dagegen sind die Anschlussleitung 50 des Widerstandselements 44 und die Anschlussleitung 51 des Widerstandselements 45 zu einem Anschlussstecker 53 geführt. Diese ist eine elektrische Leitung 54 vom Handschalter 52 zum Anschlussstecker 53 geführt.

Wie dies den Figuren 8 bis 10 ferner entnommen werden kann, ist zur zumindest teilweisen thermischen Entkopplung der beiden durch die Dochtaussparung 42 und das Widerstandselement 44 sowie die Dochtaussparung 43 und das Widerstandselement 45 gebildeten Heizeinheiten 55 bzw. 56 in einem Bereich zwischen den beiden Dochtaussparungen 42, 43 ein durch den Heizblock 40 durchgehender Luftspalt 57 als Trennmittel vorgesehen.

Vorzugsweise weisen die beiden Widerstandselemente 44, 45 unterschiedliche Widerstandwerte auf.

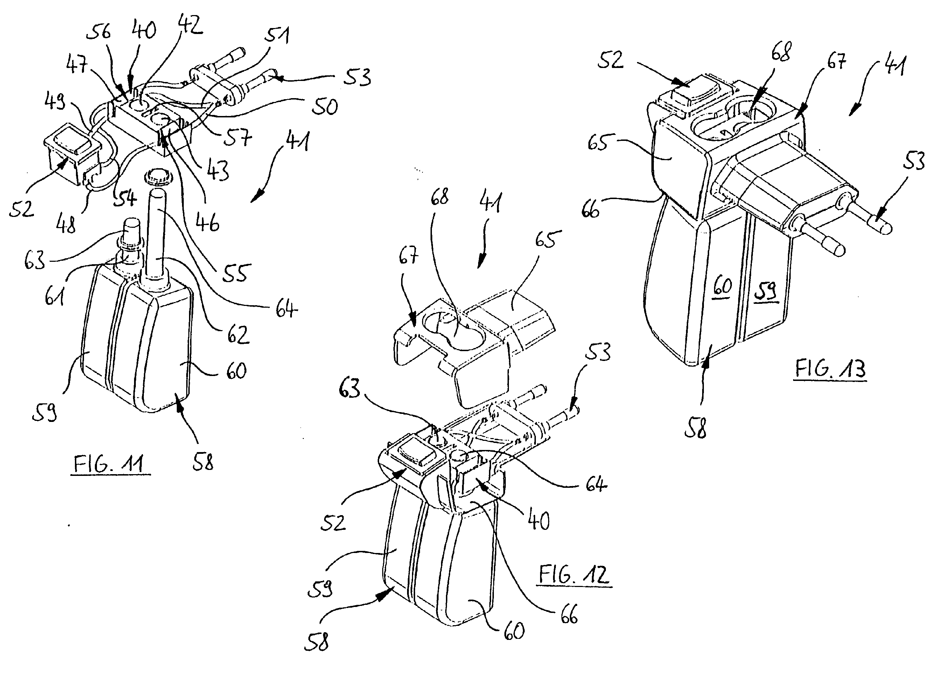

Wie dies den Figuren 11 bis 13 entnommen werden kann, weist die Verdampfungsvorrichtung 41 ferner ein Behältnis 58 mit zwei Kammern 59, 60 als Behälter auf, wobei jeder Kammer jeweils ein Docht 61, 62 zugeordnet ist, der im montierten Zustand mit einem Dochtende 63, 64 aus den beiden Kammern 59, 60 hervorsteht. In den beiden Kammern sind vorzugsweise unterschiedliche zu verdampfende Substanzen enthalten, die eine unterschiedliche Verdampfungstemperatur aufweisen.

Im montierten Zustand ist der Heizblock 40 in einem Gehäuse 67, das wiederum aus einer Gehäuse-Oberschale 65 und einer Gehäuse-Unterschale 66 aufgebaut ist, aufgenommen, wie dies insbesondere aus der Fig. 12 ersichtlich ist. An diesem Gehäuse 67 kann ferner eine Aussparung zur Integration des Handschalters 52 vorgesehen sein. Weiter umfasst die Oberschale 65 einen Lüftungsschlitz 68.

Über den Handschalter 52 können die beiden Heizeinheiten 55, 56 einzeln aktiviert werden, so dass entweder die in der Kammer 59 oder die in der Kammer 60 aufgenommene flüchtige Substanz verdampft wird. Die Widerstandselemente 44, 45 können dabei in ihrem Widerstandswert entsprechend an die zu verdampfenden Substanzen in den Kammern 59, 60 angepasst sein. Der Handschalter 52 kann zudem noch eine weitere Schaltposition aufweisen, mit der beide Heizeinheiten 55, 56 aktiviert sind, so dass, insbesondere im Rahmen einer Aromatherapie ein Duftstoffgemisch durch Verdampfen aus beiden Kammern 59, 60 erzeugt werden kann. In einer weiteren Schaltposition des Handschalters 52 sind dagegen beide Heizeinheiten 55, 56 deaktiviert. Diese Schaltstellungen des Handschalters 52 sind hier nicht im Detail dargestellt.

## Patentansprüche

1. Vorrichtung zum Verdampfen von flüchtigen Substanzen, insbesondere von Insektiziden und/oder Duftstoffen,
mit einem Gehäuse (25; 67),
mit einer im Gehäuse (25; 67) angeordneten Heizeinrichtung (1), die einen Heizblock (2; 40) umfasst, der zur Erwärmung des Heizblocks (2; 40) ein Heizelement (5; 44) aufweist,
mit einem mit dem Gehäuse (25; 67) verbindbaren Behälter (29; 59) für eine zu verdampfende Substanz, wobei in den Behälter (29; 59) ein Docht (30; 61) einsetzbar ist, der bei mit dem Gehäuse (25; 67) verbundenem Behälter (29; 59) zur Verdampfung der sich im Behälter (29; 59) befindlichen Substanz mit einem aus dem Behälter (29; 59) ragenden Dochtende (32; 63) in eine Dochtaussparung (4; 42) des Heizblocks (2; 40) ragt,
mit einer Schalteinrichtung (17; 52) zur Aktivierung und Deaktivierung der Heizeinrichtung (1), und
mit einer Einstelleinrichtung zur Einstellung des Verdampfungsgrades,
**dadurch gekennzeichnet,**
**dass** in den Heizblock (2; 40) wenigstens zwei Heizelemente (5, 6; 44, 45) eingegossen sind, mit denen unterschiedliche Heizleistungen einstellbar sind, und
**dass** die wenigstens zwei Heizelemente (5, 6; 44, 45) zu deren Aktivierung und/oder Deaktivierung mit der gleichzeitig die Einstelleinrichtung bildenden Schalteinrichtung (17; 52) gekoppelt sind.

2. Vorrichtung nach Anspruch 1, **dadurch gekennzeichnet,**
**dass** die Heizelemente (5, 6; 44, 45) so mit der Schalteinrichtung (17; 52) gekoppelt sind, dass die Heizelemente (5, 6; 44, 45) zur Einstellung des Verdampfungsgrades einzeln aktivierbar oder deaktivierbar sowie gemeinsam deaktivierbar sind, und/oder
**dass** die Heizelemente (5, 6; 44, 45) vorzugsweise auch gruppenweise oder gemeinsam aktivierbar sind.

3. Vorrichtung nach Anspruch 1 oder Anspruch 2, **dadurch gekennzeichnet, dass** die Dochtaussparung (4) bezogen auf die Draufsicht in einem mittleren Bereich zwischen zwei Heizelementen (5, 6) ausgebildet ist.

4. Vorrichtung nach Anspruch 3, **dadurch gekennzeichnet,**
**dass** der Heizblock (2) in einer Draufsicht eine rechteckige oder ovale Form aufweist, und
**dass** die Dochtaussparung (4) bezogen auf die Draufsicht in einem mittleren Bereich des Heizblocks (2) ausgebildet ist.

5. Vorrichtung nach Anspruch 3 oder Anspruch 4, **dadurch gekennzeichnet, dass** die Heizelemente (5, 6) jeweils einen gleichen Abstand zur Dochtaussparung (4) für eine symmetrische Anordnung der Heizelemente (5, 6) bezüglich der Dochtaussparung (4) aufweisen.

6. Vorrichtung nach Anspruch 1 oder Anspruch 2, **dadurch gekennzeichnet,**
**dass** wenigstens eine weitere Dochtaussparung (43) am Heizblock (40) vorgesehen ist, wobei jeder Dochtaussparung (42, 43) wenigstens ein Heizelement (44, 45) zugeordnet ist und wobei jede Dochtaussparung (42, 43) sowie jedes einer Dochtaussparung (42, 43) zugeordnete Heizelement (44, 45) jeweils eine Heizeinheit (55, 56) bilden, und
**dass** jeder weiteren Dochtaussparung (43) jeweils ein weiterer Behälter (60) mit darin einsetzbarem Docht (62) so zugeordnet ist, dass ein Dochtende (64) des Dochtes (62) des weiteren Behälters (60) zur Verdampfung der sich im weiteren Behälter (60) befindlichen Substanz in die weitere Dochtaussparung (43) hineinragt.

7. Vorrichtung nach Anspruch 6, **dadurch gekennzeichnet, dass** am Heizblock (40) zwei Dochtaussparungen (42, 43) vorgesehen sind, denen jeweils ein Heizelement (44, 45) zugeordnet ist.

8. Vorrichtung nach Anspruch 7, **dadurch gekennzeichnet, dass** zwei Dochtaussparungen (42, 43) beabstandet voneinander sowie bezogen auf eine Draufsicht auf den Heizblock (40) in einem mittleren Bereich zwischen zwei im randseitigen Bereich des Heizblocks (40) angeordneten Heizelementen (44, 45) angeordnet sind.

9. Vorrichtung nach Anspruch 7 oder Anspruch 8, **dadurch gekennzeichnet, dass** die beiden Behälter jeweils separate Behältnisse sind oder durch ein einziges Behältnis (58) mit zwei voneinander abgetrennten Kammern als Behälter (59, 60) gebildet sind, wobei in den beiden Behältern (59, 60) zu verdampfende Substanzen aufgenommen sind.

10. Vorrichtung nach einem der Ansprüche 7 bis 9, **dadurch gekennzeichnet, dass** zur zumindest teilweisen thermischen Entkopplung der beiden durch je ein Heizelement (44, 45) und eine zugeordnete Dochtaussparung (42, 43) gebildeten Heizeinheiten (55, 56) in einem Bereich zwischen den beiden Dochtaussparungen (42, 43) wenigstens ein Trennmittel (57) vorgesehen ist.

11. Vorrichtung nach Anspruch 10, **dadurch gekennzeichnet, dass** das Trennmittel (57) zur thermischen Entkopplung durch einen wenigstens im Bereich zwischen den beiden Dochtaussparungen (42, 43) durch den Heizblock (40) durchgehenden Luftspalt gebildet ist.

12. Vorrichtung nach einem der Ansprüche 1 bis 11, **dadurch gekennzeichnet, dass** jedes Heizelement (5, 6; 44, 45) durch ein elektrisches Widerstandselement gebildet ist, das in den Heizblock eingegossen ist.

13. Vorrichtung nach Anspruch 12, **dadurch gekennzeichnet, dass** die elektrischen Widerstandselemente (5, 6; 44, 45) stabförmig ausgebildet sind.

14. Vorrichtung nach Anspruch 12 oder Anspruch 13, **dadurch gekennzeichnet, dass** die Widerstandselemente (5, 6; 44, 45) zur Bereitstellung unterschiedlicher Heizleistungen unterschiedliche Widerstandswerte aufweisen.

15. Vorrichtung nach einem der Ansprüche 1 bis 14, **dadurch gekennzeichnet, dass** jedes Heizelement (5, 6; 44, 45) über elektrische Leitungen (13, 14, 15, 16; 48, 49, 50, 51) mit einem am Gehäuse (25; 67) angeordneten Anschlussstecker (18; 53) und der ebenfalls am Gehäuse (25; 67) angeordneten Schalteinrichtung (17; 52) gekoppelt ist.

16. Vorrichtung nach einem der Ansprüche 12 bis 15, **dadurch gekennzeichnet, dass** jedes elektrische Widerstandselement (5, 6; 44, 45) einen stabförmigen Widerstandskörper (20) aufweist, der wenigstens bereichsweise mit einer Widerstandsschicht (21) beschichtet ist, die zur Einstellung eines bestimmten Widerstandswertes entsprechend einer auf die Zusammensetzung der jeweils zu verdampfenden Substanz abgestimmten Verdampfungstemperatur bereichsweise eingeschnitten und/oder bereichsweise eingeschliffen ist.

17. Vorrichtung nach Anspruch 16, **dadurch gekennzeichnet, dass** die Widerstandsschicht (21) um den stabförmigen Widerstandskörper (20) herum spiralförmig mit einem Spiralschnitt (22) eingeschnitten ist.

18. Vorrichtung nach Anspruch 16 oder Anspruch 17, **dadurch gekennzeichnet,**
**dass** das stabförmige Widerstandselement (5, 6; 44, 45) in eine Aussparung (7, 8; 46, 47) des Heizblocks (2; 40) einbringbar ist und dort mit einem eine hohe Wärmeleitfähigkeit aufweisenden Material vergossen ist, und
**dass** an gegenüberliegenden Aussparungswänden zu beiden Seiten des Widerstandselementes (5, 6; 44, 45) jeweils ein Schlitz (13, 14, 15, 16) ausgebildet ist, durch die die elektrischen Leitungen (13, 14, 15, 16; 48, 49, 50, 51) aus dem Heizblock (2; 40) zu einem Anschlussstecker (25; 67) bzw. zur Schalteinrichtung (17; 53) hin herausgeführt sind.

19. Vorrichtung nach einem der Ansprüche 1 bis 18, **dadurch gekennzeichnet, dass** die Dochtaussparung (4; 42, 43) als Durchgangsloch ausgebildet ist.

20. Vorrichtung nach einem der Ansprüche 1 bis 19, **dadurch gekennzeichnet, dass** die Schalteinrichtung durch einen Handschalter (17; 52) oder durch einen programmierbaren Mikroprozessor gebildet ist.

21. Vorrichtung nach einem der Ansprüche 1 bis 20, **dadurch gekennzeichnet,**
**dass** das Gehäuse (25; 67) wenigstens zweiteilig aus einer Oberschale (27; 65) und einer Unterschale (26; 66) aufgebaut ist,
**dass** die Unterschale (26; 66) Verbindungsmittel zur Verbindung des Behälters (29; 59, 60) mit dem Gehäuse (25; 67) aufweist, und
**dass** wenigstens eine der beiden Schalen (25, 26; 65, 66) wenigstens einen Lüftungsschlitz (28; 68) zum Entweichen der verdampften Substanzen aufweist.

## Claims

1. Device for vaporizing volatile substances, in particular insecticides and/or perfumes,
with a housing (25; 67),
with a heating arrangement (1) which is disposed in the housing (25; 67) and comprises a heating block (2; 40) which has a heating element (5; 44) for the purpose of warming the heating block (2; 40),
with a container (29; 59) for a substance to be vaporized, which container can be connected to the housing (25; 67) and into which container (29; 59) it is possible to insert a wick (30; 61) which, when the container (29; 59) is connected to the housing (25; 67), protrudes into a wick recess (4; 42) of the heating block (2; 40) with a wick end (32; 63) protruding from the container (29; 59) in order to vaporize the substance located in the container (29; 59),
with a switch mechanism (17; 52) for activation and deactivation of the heating arrangement (1), and
with an adjustment mechanism for adjusting the degree of vaporization,
**characterized in that**
at least two heating elements (5, 6; 44, 45), with which different heating capacities can be set, are cast into the heating block (2; 40), and
the at least two heating elements (5, 6; 44, 45) are coupled, for their activation and/or deactivation, to the switch mechanism (17; 52), which at the same time forms the adjustment mechanism.

2. Device according to Claim 1, **characterized in that**
the heating elements (5, 6; 44, 45) are coupled to the switch mechanism (17; 52) in such a way that the heating elements (5, 6; 44, 45) can be individually activated or deactivated and can be jointly deactivated in order to adjust the degree of vaporization, and/or **in that**
the heating elements (5, 6; 44, 45) can preferably also be activated in groups or jointly.

3. Device according to Claim 1 or Claim 2, **characterized in that**, relative to the plan view, the wick recess (4) is formed in a central area between two heating elements (5, 6).

4. Device according to Claim 3, **characterized in that**
the heating block (2) has a rectangular or oval shape in a plan view, and
the wick recess (4), relative to the plan view, is formed in a central area of the heating block (2).

5. Device according to Claim 3 or Claim 4,
**characterized in that** the heating elements (5, 6) are each spaced at equal distances from the wick recess (4) to ensure a symmetrical arrangement of the heating elements (5, 6) relative to the wick recess (4).

6. Device according to Claim 1 or Claim 2, **characterized in that**
at least one further wick recess (43) is provided in the heating block (40), each wick recess (42, 43) being assigned at least one heating element (44, 45), and each wick recess (42, 43) and each heating element (44, 45) assigned to a wick recess (42, 43) forming a heating unit (55, 56), and
each further wick recess (43) is assigned a further container (60) with a wick (62) that can be inserted therein such that a wick end (64) of the wick (62) of the further container (60) protrudes into the further wick recess (43) for vaporization of the substance located in the further container (60).

7. Device according to Claim 6, **characterized in that** the heating block (40) is provided with two wick recesses (42, 43) to each of which a respective heating element (44, 45) is assigned.

8. Device according to Claim 7, **characterized in that** two wick recesses (42, 43) are spaced apart from one another and, relative to a plan view of the heating block (40), are arranged in a central area between two heating elements (44, 45) that are arranged in the area of the heating block (40) near its edge.

9. Device according to Claim 7 or Claim 8, **characterized in that** the two containers are each separate receptacles or are formed by a single receptacle (58) having two separate chambers as containers (59, 60), and substances to be vaporized are received in both containers (59, 60).

10. Device according to one of Claims 7 to 9, **characterized in that** at least one separator (57) is provided in an area between the two wick recesses (42, 43) in order to achieve at least partial thermal uncoupling of the two heating units (55, 56) formed in each case by a heating element (44, 45) and an associated wick recess (42, 43).

11. Device according to Claim 10, **characterized in that** the separator (57) for the thermal uncoupling is formed by an air gap that extends through the heating block (40) at least in the area between the two wick recesses (42, 43).

12. Device according to one of Claims 1 to 11, **characterized in that** each heating element (5, 6; 44, 45) is formed by an electrical resistance element that is cast into the heating block.

13. Device according to Claim 12, **characterized in that** the electrical resistance elements (5, 6; 44, 45) are rod-shaped.

14. Device according to Claim 12 or Claim 13, **characterized in that** the resistance elements (5, 6; 44, 45) have different resistance values in order to provide different heating capacities.

15. Device according to one of Claims 1 to 14, **characterized in that** each heating element (5, 6; 44, 45) is coupled via electrical lines (13, 14, 15, 16; 48, 49, 50, 51) to a connector plug (18; 53) arranged on the housing (25; 67) and to the switch mechanism (17; 52) also arranged on the housing (25; 67).

16. Device according to one of Claims 12 to 15, **characterized in that** each electrical resistance element (5, 6; 44, 45) has a rod-shaped resistance body (20) which is coated at least in some areas with a resistance layer (21) which, in order to set a defined resistance value corresponding to a vaporization temperature for the composition of the respective substance to be vaporized, is notched in some areas and/or ground-in in some areas.

17. Device according to Claim 16, **characterized in that** the resistance layer (21) around the rod-shaped resistance body (20) is notched in a helical formation with a helical cut (22).

18. Device according to Claim 16 or Claim 17, **characterized in that**
the rod-shaped resistance element (5, 6; 44, 45) can be introduced into a recess (7, 8; 46, 47) of the heating block (2; 40) and is there encapsulated with a material having high thermal conductivity, and,
at opposite recess walls, on both sides of the resistance element (5, 6; 44, 45), a slit (13, 14, 15, 16) is in each case formed through which the electrical lines (13, 14, 15, 16; 48, 49, 50, 51) are routed from the heating block (2; 40) to a connector plug (25; 67) or to the switch mechanism (17; 53).

19. Device according to one of Claims 1 to 18, **characterized in that** the wick recess (4; 42, 43) is designed as a through-hole.

20. Device according to one of Claims 1 to 19, **characterized in that** the switch mechanism is formed by a manual switch (17; 52) or by a programmable microprocessor.

21. Device according to one of Claims 1 to 20, **characterized in that**
the housing (25; 67) is made up of at least two parts, namely an upper shell (27; 65) and a lower shell (26; 66),
the lower shell (26; 66) has connector means for connecting the container (29; 59, 60) to the housing (25; 67), and
at least one of the two shells (25, 26; 65, 66) has at least one ventilation slot (28; 68) for escape of the vaporized substance.

## Revendications

1. Dispositif de vaporisation de substances liquides, notamment insecticides et / ou parfums,
avec un boîtier (25 ; 67)
avec un système de chauffe (1) disposé dans le boîtier (25, 67), qui comprend un bloc chauffant (2 ; 40) présentant un élément de chauffe (5; 44) pour le réchauffement du bloc chauffant (2 ; 40),
avec un récipient (29 ; 59) raccordable au boîtier (25 ; 67) pour une substance devant être vaporisée, à l'occasion de quoi une mèche (30 ; 61) est insérée dans le récipient (29 ; 59) et sort du récipient (29 ; 59) relié au boîtier (25 ; 67) pour la vaporisation de la substance se trouvant dans le récipient (29 ; 59) avec une extrémité de mèche (32 ; 63) en saillie du récipient (29 ;59) dans un creux destiné à la mèche (4 ; 42) du bloc chauffant (2 ; 40),
avec un dispositif d'actionnement (17 ; 52) pour l'activation et la désactivation du système de chauffe (1), et
avec un système de réglage pour le réglage du niveau de vaporisation,
**caractérisé en ce que**
au moins deux éléments de chauffe (5, 6 ; 44, 45) sont coulés dans le bloc chauffant (2 ; 40), grâce auxquels différentes prestations de chauffe sont ajustables et
**en ce que**, pour leur activation et / ou désactivation, au moins les deux éléments de chauffe (5, 6 ; 44, 45) sont accouplés avec le dispositif d'actionnement (17 ; 52) formant simultanément le système de réglage.

2. Dispositif conformément à la revendication 1, **caractérisé en ce que**
les éléments de chauffe (5, 6 ; 44, 45) sont accouplés avec le dispositif d'actionnement (17 ; 52) de sorte que les éléments de chauffe (5, 6; 44, 45) sont activables ou désactivables individuellement pour le réglage du niveau de vaporisation ainsi que désactivables ensemble et / ou
**en ce que** les éléments de chauffe (5, 6 ; 44, 45) sont activables de préférence par groupe ou ensemble.

3. Dispositif conformément à la revendication 1 ou 2, **caractérisé en ce que** le creux destiné à la mèche (4), par rapport à la vue de dessus, est formé dans une zone centrale entre deux éléments de chauffe (5, 6).

4. Dispositif conformément à la revendication 3, **caractérisé en ce que**
que le bloc chauffant (2), en vue de dessus, présente une forme rectangulaire ou ovale, et
**en ce que** le creux destiné à la mèche (4) par rapport à la vue de dessus est formé dans une zone centrale du bloc chauffant (2).

5. Dispositif conformément à la revendication 3 ou 4, **caractérisé en ce que** les éléments de chauffe (5, 6) présentent respectivement la même distance par rapport au creux destiné à la mèche (4) pour une disposition symétrique des éléments de chauffe (5, 6) par rapport au creux destiné à la mèche (4).

6. Dispositif conformément à la revendication 1 ou 2, **caractérisé en ce que**
au moins une autre creux destiné à la mèche (43) est prévu sur le bloc chauffant (40), au moins un élément de chauffe (44, 45) étant affecté à chaque creux destiné à la mèche (42, 43) et chaque creux destiné à la mèche (42, 43) ainsi que chaque élément de chauffe (44, 45) affecté à un creux destiné à la mèche (42, 43) formant respectivement une unité de chauffe (55 , 56) et
**en ce que** respectivement un autre récipient (60) avec une mèche (62) insérable dedans est affecté à chaque nouveau creux destiné à la mèche (43), de sorte qu'une extrémité (64) de la mèche (62) du récipient supplémentaire (60), pour la vaporisation de la substance se trouvant dans le récipient supplémentaire (60), pénètre dans l'autre creux destiné à la mèche (43).

7. Dispositif conformément à la revendication 6, **caractérisé en ce que** sur le bloc chauffant (40) deux creux destinés à la mèche (42, 43) sont prévus, auxquels il est respectivement affecté un élément de chauffe (44 , 45).

8. Dispositif conformément à la revendication 7, **caractérisé en ce que** deux creux destinés à la mèche (42, 43) sont disposés à distance l'un de l'autre ainsi que, par rapport à une vue de dessus, sur le bloc chauffant (40) dans une zone centrale entre deux éléments de chauffe (44, 45) disposés dans la zone marginale du bloc chauffant (40).

9. Dispositif conformément à la revendication 7 ou 8, **caractérisé en ce que** les deux récipients sont des récipients respectivement séparés ou sont formés, en tant que récipient (59,60) par un seul récipient (58) avec deux chambres séparées l'une de l'autre, des substances devant être vaporisées étant logées dans les deux récipients (59 ,60).

10. Dispositif conformément à l'une des revendications 7 à 9, **caractérisé en ce que**, pour un découplage au moins partiellement thermique des deux unités de chauffe (55, 56) formées respectivement par un élément de chauffe (44, 45) et un creux destiné à la mèche (42, 43) associé, au moins un moyen de séparation (57) est prévu dans une zone entre les deux creux destinés à la mèche (42 ,43)

11. Dispositif conformément à la revendication 10, **caractérisé en ce que** le moyen de séparation (57) pour le découplage thermique est formé par une fente traversée au moins dans le secteur entre les deux creux destinés à la mèche (42, 43) par le bloc chauffant (40).

12. Dispositif conformément à l'une des revendications 1 à 11, **caractérisé en ce que** chaque élément de chauffe (5, 6 ; 44, 45) est formé par un élément de résistance électrique, qui est coulé dans le bloc chauffant.

13. Dispositif conformément à la revendication 12, **caractérisé en ce que** les éléments de résistance électriques (5, 6 ; 44, 45) sont développés sous forme de barre.

14. Dispositif conformément à la revendication 12 ou 13, **caractérisé en ce que** les éléments de résistance électriques (5, 6 ; 44, 45) présentent différentes valeurs de résistance pour la mise à disposition de différentes prestations de chauffe.

15. Dispositif conformément à l'une des revendications 1 à 14, **caractérisé en ce que** chaque élément de chauffe (5, 6 ; 44, 45) est couplé via des lignes électriques (13, 14, 15, 16 ; 48, 49, 50 ,51) avec une fiche de raccordement (18 ; 53) disposée sur le boîtier (25 ; 67) et le dispositif de commutation (17 ; 52) également disposé sur le boîtier (25 ; 67).

16. Dispositif conformément à l'une des revendications 12 à 15, **caractérisé en ce que** chaque élément de résistance électrique (5, 6 ; 44, 45) présente un corps de résistance (20) en forme de barre, qui est revêtu au moins partiellement d'une couche résistive (21), qui est entaillée et / ou rodée partiellement pour le réglage d'une valeur de résistance déterminée conformément à une température de vaporisation adaptée à la composition de la substance respective devant être vaporisée.

17. Dispositif conformément à la revendication 16, **caractérisé en ce que** la couche résistive (21) est entaillée avec une coupe en forme de spirale (22) formant une forme de spirale autour du corps de résistance en forme de barre (20).

18. Dispositif conformément à la revendication 16 ou 17, **caractérisé en ce que**
l'élément de résistance électrique en forme de barre (5, 6 ; 44, 45) peut être placé dans un creux (7, 8 ; 46, 47) du bloc chauffant (2 ; 40) et y est coulé avec un matériau présentant une grande conductibilité thermique et
**en ce qu'**une fente (13, 14, 15, 16) est respectivement formée sur les parois opposées du creux, des deux côtés de l'élément de résistance électrique (5, 6 ; 44, 45), fentes à travers lesquelles sont sorties les lignes électriques (13, 14, 15, 16 ; 48, 49, 50 ,51) du bloc chauffant (2 ; 40) vers une fiche de raccordement (25 ; 67) ou selon le cas vers le dispositif de commutation (17 ; 53).

19. Dispositif conformément à l'une des revendications 1 à 18, **caractérisé en ce que** le creux destiné à la mèche (4 ; 42, 43) est réalisé sous forme de trou de passage.

20. Dispositif conformément à l'une des revendications 1 à 19, **caractérisé en ce que** le dispositif de commutation est formé par un commutateur à commande manuelle (17 ; 52) ou par un microprocesseur programmable .

21. Dispositif conformément à l'une des revendications 1 à 20, **caractérisé en ce que** le
le boîtier (25 ; 67) est constitué d'au moins deux parties composées d'une coquille supérieure (27 ; 65) et d'une coquille inférieure (26 ; 66),
**en ce que** la coquille inférieure (26 ; 66) présente des moyens de raccordement pour le raccordement du récipient (29 ; 59, 60) avec le boîtier (25 ; 67) et
**en ce qu'**au moins l'une des deux coquilles (25, 26 ; 65, 66) présente au moins une fente d'aération (28 ; 68) pour dégager les substances vaporisées.
